# EUROPEAN PATENT APPLICATION

(11) **EP 2 607 487 A1**
(43) Date of publication of application: **26.06.2013**
(21) Application number: 11195715.5
(22) Date of filing: 23.12.2011
(51) Int. Cl.: C12N 15/82, A01H 5/00, A01H 5/12

(54) **Resistance against Peronospora parasitica in Diplotaxis tenuifolia**

(71) Applicant: Rijk Zwaan Zaadteelt en Zaadhandel B.V., 2678 KX De Lier (NL)
(72) Inventor: Carree, Franciscus Hermanus, 4759 CB Noordhoek (NL)
(74) Representative: Van Someren, Petronella F. H. M.

(57) **Abstract**

The invention relates to a rucola plant of the species *Diplotaxis tenuifolia* comprising a genetic determinant that leads to expression of resistance against *Peronospora parasitica,* which genetic determinant is obtainable from a *Diplotaxis tenuifolia* plant comprising said genetic determinant, representative seed of which was deposited with the NCIMB under deposit number NCIMB 41811. The invention further provides seeds that can grow into a plant that is resistant to *Peronospora parasitica,* to progeny of the rucola plant to propagation material suitable for producing the plant or seed and to parts of the plant and food products comprising the plant parts.

## Description

The present invention relates to a Diplotaxis tenuifolia plant comprising a genetic determinant that leads to expression of resistance against Peronospora parasitica. The invention further relates to a source of resistance to Peronospora parasitica for use in breeding. The invention also relates to the seeds and progeny of such plants and to propagation material for obtaining such plants. Furthermore the invention relates to the use of the plants, seeds and propagation material that comprises the genetic determinant as germplasm in a breeding program.

Diplotaxis tenuifolia is an edible plant species and member of the Brassicaceae, the mustard plant family, known for numerous other edible plants such as Sinapis alba (mustard), Brassica oleracea (e.g. broccoli, cabbage, cauliflower), Brassica rapa (e.g. turnip, Chinese cabbage), Brassica napus (e.g. rapeseed), Raphanus sativus (common radish), Armorica rusticana (horseradish) and many others. Other names for Diplotaxis tenuifolia include rucola, wild rocket, or just rocket. The plant is often confused with another Brassicaceae member, Eruca sativa, which looks very similar, is used for the same purposes, and is also called rucola, salad rocket, or arugula. E. sativa however is a different species that cannot be crossed with D. tenuifolia.

D.tenuifolia is a diploid and perennial species, native to Europe and Western Asia. It can be found throughout much of the temperate world where it has been naturalized. It is an erect mustard-like plant with branching stems that may exceed half a meter in height. It grows in clumps on the ground in a variety of habitats and is a common weed of roadsides and disturbed areas. It has long leaves which may be lobed or not. The foliage is aromatic when crushed. Atop the branches of the stem are bright yellow flowers with four rounded petals each about a centimeter long. The fruit is a straight, flat silique up to five centimeters long.

All over the world Diplotaxis tenuifolia is grown as a salad vegetable. Especially in Italy, D. tenuifolia is an important crop. In other countries this crop has the potential to become more important over the coming years. The use of the young leaves of these plants is traditional in the Mediterranean cuisine (Gilardi et al., 2007, D'Antuono et al., 2008). Because of the popular taste, the low amount of calories, and decorative effect, rocket salad is now very often appearing in many other dishes.

In Europe as well as in many other areas the production of rucola is being hindered by the infection of the plants by downy mildew (Peronospora parasitica). Downy mildew is a polycyclic disease caused by different species of Hyaloperonospora and Peronospora (Goker et al., 2009). The obligate parasite from the genus Hyaloperonospora and the genus Peronospora is living on Brassica plants and closely related cruciferous crops. The losses are more severe at the seedling stage than on mature healthy plants (Koike et al., 2007, Rimmer et al., 2007). Young seedlings can die as a result of the infection (Rimmer et al., 2007). Cool and moist conditions are favourable for the disease development (Smith et al., 1988). The disease causes quantitative and qualitative losses of the crops. Although all (aerial) parts of the plant can be infected, the symptoms primarily appear on the leaves (Rimmer et al., 2007). Symptoms of the disease such as discoloured lesions and necrotic spots with brown edges can destroy the quality of the leaves. Also the quantity of the harvest is reduced due to the downy mildew infection.

All varieties currently available on the market are susceptible to downy mildew. Gene bank accessions were tested during internal research but no resistance was identified.

During the research that led to the present invention a new Diplotaxis tenuifolia plant was created that exhibits resistance against downy mildew. Several sources were used to create this plant, all of them showing susceptibility for downy mildew. Surprisingly, rucola material with a high level of resistance against Peronospora parasitica, the downy mildew that infects rucola, was developed on the basis of these susceptible sources.

It is thus an object of the present invention to provide a Diplotaxis tenuifolia plant that is resistant to Peronospora parasitica.

The present invention thus provides a Diplotaxis tenuifolia plant comprising a genetic determinant which leads to the expression of resistance against Peronospora parasitica, which genetic determinant is obtainable from a Diplotaxis tenuifolia plant comprising said genetic determinant, representative seed of which was deposited with the NCIMB under deposit number NCIMB 41811.

In one embodiment, the rucola plant of the invention carrying the genetic determinant is obtainable by crossing a first rucola plant with a second rucola plant, wherein one of the said plants is grown from seed as deposited with the NCIMB under deposit number NCIMB 41811, or a progeny plant thereof, and selecting for a plant that shows resistance to Peronospora parasitica.

Selection for a plant that shows resistance to downy mildew is preferably done in the F2.

The resistance to downy mildew (Peronospora parasitica) is preferably a complete resistance. When the plants are tested for downy mildew in a bio-assay, they are completely free from downy mildew symptoms. Several bio-assays were carried out to confirm the high level of resistance. Seeds from rucola plants that have complete resistance against downy mildew were deposited with the NCIMB under deposit number NCIMB 41811.

It was found that the resistance to downy mildew of the invention is inherited in a dominant way.

In a preferred embodiment, the resistance against Peronospora parasitica is transmitted by dominant inheritance when the plant that comprises the genetic determinant is used as the female parent in a cross. The resulting F1 from a cross between a resistant Diplotaxis tenuifolia plant of the invention and a susceptible D. tenuifolia plant is completely resistant. In the F2 the resistance segregates and resistant plants can be selected.

Selection of completely resistant plants can be done by performing a bio-assay under controlled conditions that favour the development of downy mildew on the rucola plants.

For obtaining a resistant hybrid of rucola, the line used as the female preferably comprises the genetic determinant of the invention that leads to resistance to downy mildew.

In one embodiment, the genetic determinant that leads to resistance against downy mildew is obtainable by introgression from a rucola plant, representative seed of which has been deposited with the NCIMB under deposit number NCIMB 41811.

"Introgression" as used herein is intended to mean introduction of a trait into a plant not carrying the trait by means of crossing and selection in the first generation in which the trait becomes visible. Selection can alternatively start in any subsequent generation in which the trait is visible or segregates.

The invention furthermore relates to a cell of a rucola plant as claimed. Such cell may be either in isolated form or may be part of the complete rucola plant or parts thereof and then still constitutes a cell of the invention because such a cell harbours in its genetic constitution the genetic information that leads to the characteristics that define the rucola plant of the invention. Each cell of rucola plants of the invention carries the genetic information that leads to phenotypic expression of said trait.

The invention also relates to tissue of a plant as claimed. The tissue can be undifferentiated tissue or already differentiated tissue. Undifferentiated tissues are for example stem tips, anthers, petals, pollen and can be used in micropropagation to obtain new plantlets that are grown into new plants of the invention.

The invention according to a further aspect thereof relates to seeds of a plant as claimed. Although the seeds do not show the genetic trait of the rucola plant of the invention, they harbour the genetic information that when a plant is grown from the seeds makes this plant a plant of the invention. The invention also relates to seed comprising the genetic determinant of the invention, wherein the plant that can be grown from the seed is resistant to Peronospora parasitica.

The invention also relates to progeny of the plants, cells, tissues and seeds of the invention. Such progeny can in itself be plants, cells, tissues or seeds. The invention relates to progeny of a rucola plant of the invention, or of rucola seed that harbours the trait of the invention, which progeny is resistant to Peronospora parasitica.

As used herein the word "progeny" is intended to mean the first and all further descendants from a cross with a plant of the invention that comprises a genetic determinant that leads to complete resistance against Peronospora parasitica. Progeny of the invention are descendants of any cross with a plant of the invention that carries the trait that leads to resistance against downy mildew. "Progeny" also encompasses plants that carry the trait of the invention and are obtained from other plants or progeny of plants of the invention by vegetative propagation or multiplication.

The invention thus further relates to seed of the claimed plant and to parts of the plant that are suitable for sexual reproduction. Such parts are for example selected from the group consisting of microspores, pollen, ovaries, ovules, embryo sacs and egg cells. In addition, the invention relates to parts of the plant that are suitable for vegetative reproduction, in particular cuttings, roots, stems, cells, and protoplasts.

According to a further aspect thereof the invention provides a tissue culture of the claimed plant. The tissue culture suitably comprises regenerable cells. Such tissue culture can be derived from leaves, pollen, embryos, cotyledons, hypocotyls, meristematic cells, roots, root tips, anthers, flowers, seeds and stems.

The invention furthermore relates to hybrid seed and to a method for producing hybrid seed comprising crossing a first parent plant with a second parent plant and harvesting the resultant hybrid seed, wherein said first parent plant and/or said second parent plant is the plant as claimed.

The invention also relates to inbreds and doubled haploids of rucola plants of the invention.

In one embodiment, the invention relates to rucola plants of the invention that carry the genetic determinant which leads to resistance against P. parasitica, and having acquired said determinant by introduction of the genetic information that is responsible for the trait from a suitable source, either by conventional breeding, or genetic modification, in particular by cisgenesis or transgenesis. Cisgenesis is genetic modification of plants with a natural gene, coding for an (agricultural) trait, from the crop plant itself or from a sexually compatible donor plant. Transgenesis is genetic modification of a plant with a gene from a non-crossable species or a synthetic gene.

In one embodiment, the invention relates to a method for production of a Diplotaxis tenuifolia plant comprising resistance to Peronospora parasitica, comprising
a) crossing a plant comprising the genetic determinant that leads to expression of Peronospora parasitica resistance with another plant;
b) selfing the resulting F1 for obtaining F2 plants;
c) selecting resistant plants in the F2; and
d) optionally performing one or more additional rounds of selfing or crossing, and subsequently selecting, for a plant comprising resistance against downy mildew.

It is clear that the parent that provides the trait of the invention is not necessarily a plant grown directly from the deposited seeds. The parent can also be a progeny plant from the seed or a progeny plant from seeds that are identified to have the trait of the invention by other means.

In one embodiment, the invention relates to a method for production of a Diplotaxis tenuifolia plant comprising resistance to Peronospora parasitica, comprising:
a) crossing a plant comprising the genetic determinant that leads to expression of Peronospora parasitica resistance with another plant;
b) optionally backcrossing the resulting F1 with the preferred parent;
c) selecting resistant plants in the F2; and
d) optionally performing one or more additional rounds of selfing or crossing, and subsequently selecting, for a plant comprising resistance against downy mildew.

The invention additionally provides a method of introducing a desired trait into a rucola plant comprising resistance against downy mildew, comprising:
a) crossing a Diplotaxis tenuifolia plant comprising resistance to Peronospora parasitica, representative seed of which were deposited with the NCIMB under deposit number NCIMB 41811, with a second rucola plant that comprises a desired trait to produce F1 progeny;
b) selecting an F1 progeny that comprises resistance to Peronospora parasitica and the desired trait;
c) crossing the selected F1 progeny with either parent, to produce backcross progeny;
d) selecting backcross progeny comprising the desired trait and the resistance against Peronospora parasitica; and
e) optionally repeating steps (c) and (d) one or more times in succession to produce selected fourth or higher backcross progeny that comprises the desired trait and resistance against downy mildew. The invention, of course, includes a rucola plant produced by this method.

In one embodiment selection for resistant plants is done in the F1.

In one embodiment selection for resistant plants is started in the F3 or a later generation.

In one embodiment the plant comprising the genetic determinant is a plant of an inbred line, a hybrid, a doubled haploid, or of a segregating population.

In one embodiment, the invention relates to a method for the production of a Diplotaxis tenuifolia plant comprising resistance to Peronospora parasitica by using a doubled haploid generation technique to generate a doubled haploid line comprising the said resistance.

In one embodiment, the invention relates to a method for producing a hybrid Diplotaxis tenuifolia plant comprising crossing a first parent rucola plant with a second parent rucola plant and harvesting the resultant hybrid rucola seed, in which the first parent rucola plant and/or the second parent rucola plant comprises resistance against Peronospora parasitica.

The invention also relates to a method for the production of a rucola plant comprising resistance against Peronospora parasitica by using a seed that comprises downy mildew resistance in its genome for growing the said rucola plant.

The invention also relates to a method for seed production comprising growing plants from seeds of which a representative sample was deposited with the NCIMB under deposit number NCIMB 41811, allowing the plants to produce seeds, and harvesting those seeds. Production of the seeds is suitably done by crossing or selfing.

In one embodiment, the invention relates to a method for the production of a Diplotaxis tenuifolia plant comprising resistance against Peronospora parasitica by using tissue culture. The invention furthermore relates to a method for the production of a Diplotaxis tenuifolia plant comprising resistance against Peronospora parasitica by using vegetative reproduction.

In one embodiment, the invention relates to a method for the production of a Diplotaxis tenuifolia plant comprising resistance against Peronospora parasitica by using a method for genetic modification to introgress the resistance into the rucola plant. Genetic modification comprises transgenic modification or transgenesis, using a gene from a non-crossable species or a synthetic gene, and cisgenic modification or cisgenesis, using a natural gene, coding for an (agricultural) trait, from the crop plant itself or from a sexually compatible donor plant.

The invention also relates to the germplasm of plants of the invention. The germplasm is constituted by all inherited characteristics of an organism and according to the invention encompasses at least the trait of the invention. The germplasm can be used in a breeding program for the development of rucola plants that are resistant against downy mildew. The invention also relates to a breeding method for the development of Diplotaxis tenuifolia plants that are resistant againt Peronospora parasitica wherein germplasm comprising Peronospora parasitica resistance is used. Representative seed of said plant comprising the genetic determinant was deposited with the NCIMB under deposit number NCIMB 41811.

The invention also relates to plant parts, in particular leaves and stems, which are produced by a plant of the invention. The invention further relates to a food product, comprising the leaves and/or stems of a rucola plant as claimed, or parts thereof. The invention also relates to a food product in processed form.

In one embodiment the invention relates to a method for the production of a Diplotaxis tenuifolia plant comprising resistance against Peronospora parasitica wherein progeny or propagation material of a plant comprising the genetic determinant conferring said resistance is used as a source to introgress the resistance into another rucola plant. Representative seed of said plant comprising the genetic determinant was deposited with the NCIMB under deposit number NCIMB 41811.

The term genetic determinant' as used herein encompasses one or more genes or alleles. These terms are used interchangeably.

The genetic trait' is the trait or characteristic that is conferred by the genetic determinant. The genetic trait can be identified phenotypically, for example by performing a bio-assay. However, also plant stages for which no phenotypic assay can be performed do carry the genetic information that leads to the genetic trait. 'Trait' or 'phenotypic trait' can be used instead of 'genetic trait'.

In the absence of molecular markers, equivalence of genetic determinants can be determined by an allelism test. To perform an allelism test, material that is homozygous for the known determinant is crossed with material that is homozygous for the phenotypic trait to be tested. When no segregation for the trait to be observed is present in the F2 of the cross, the genetic determinants resulting in the phenotypic trait have been proven to be the same.

When more than one gene is responsible for a certain trait, and an allelism test is done to determine equivalence, the skilled person doing the test has to ascertain that all relevant genes are present homozygously for the test to work properly.

To determine the presence of a resistance, a bio-assay can be carried out. Bio-assays can usually be performed in several ways, as known by persons skilled in the art. One way of performing the bio-assay for determining resistance of rucola to downy mildew is described in Example 2.

The resistance of the invention as mentioned herein is a complete or high resistance. Complete resistance is expressed by symptomless plants, when these plants are exposed to a normal dose of the pathogen under common favourable conditions. This is for example the case when the bio-assay as described in Example 2 is carried out. The terms resistance, complete resistance, and high resistance can be used interchangeably.

As used throughout this application, rucola encompasses only Diplotaxis tenuifolia plants, and does not include Eruca sativa plants. The terms rucola and D. tenuifolia can be used interchangeably.

As used throughout this application, downy mildew resistance encompasses resistance against Peronospora parasitica. The terms downy mildew and Peronospora parasitica can be used interchangeably.

### DEPOSIT

Seeds of Diplotaxis tenuifolia 11.87212 that comprise the genetic determinant of the invention which leads to resistance against Peronospora parasitica were deposited with NCIMB Ltd, Ferguson Building, Craibstone 5 Estate, Bucksburn, Aberdeen AB21 9YA, UK on 16 February 2011 under deposit accession number NCIMB 41811.

### EXAMPLES

### EXAMPLE 1

### Creation of Diplotaxis tenuifolia plants of the invention

To obtain resistance against downy mildew, a variation of germplasm from the breeding program was screened through the performance of bio-assays. No resistant germplasm could be identified.

The performance of bio-assays on offspring of combinations of germplasm that were highly susceptible however resulted in the identification of some plants with resistance to Peronospora parasitica. Continued inbreeding and screening for the relevant trait led to the creation of a highly resistant D. tenuifolia plant. The high level of resistance, or complete resistance, was confirmed to be stable by the performance of a bio-assay screen on three subsequent generations. No segregation for the trait of the invention was observed. After 3 generations the seed was harvested and deposited under accession number NCIMB 41811.

Without wishing to be bound by theory, it is thought that in the sources used for creation of plants of the invention two or more genetic determinants were separately present that only resulted in resistance against downy mildew when they were combined. The combination of those genetic determinants into one background subsequently resulted in the inheritance of the phenotypic trait as if caused by one single dominant gene. It is conceivable that the two or more genetic determinants are closely linked.

### EXAMPLE 2

### Bio-assay for Peronospora parasitica.

To test whether a plant is resistant to Peronospora parasitica a bio-assay is performed. The bio-assay can be done in the following way.

Seeds are sown, for example in 4 cm peat potting blocks. The plants are grown for 2 weeks at a temperature regime of 14/12 degrees Celsius day/night. A relevant number of plants per line are evaluated, e.g. 30 plants, so that segregation can be observed if it would be present. After 2 weeks the young plants are inoculated with spores of Peronospora parasitica. The plants are scored for infection 10 days after inoculation, and again at 17 days after inoculation. Plants without any symptoms of downy mildew are completely or highly resistant.

### EXAMPLE 3

### Transfer of the trait of the invention to other rucola plants.

Resistant rucola plants that were created as described in Example 1 were used to develop other resistant rucola plants. A highly resistant D. tenuifolia plant was crossed with a susceptible D. tenuifolia plant. Unexpectedly, the resulting heterozygous F1 was highly resistant to P. parasitica. This result indicates dominant inheritance of the trait of the invention.

Subsequently highly resistant F1 plants were selfed and F2 plants were obtained. In the F2 74% resistant plants could be identified, and 26% of the plants showed symptoms of downy mildew after inoculation (Table). This further confirms the dominant inheritance of the genetic determinant of the invention. The F2 segregation ratio also establishes that the resistance to P. parasitica is conferred by a monogenetic dominant determinant.

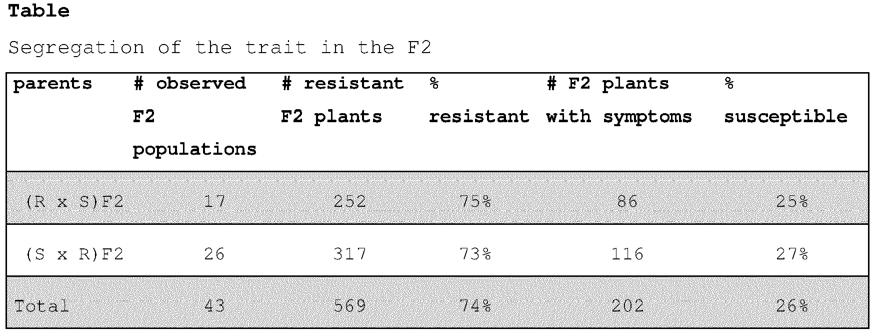

## Claims

1. Rucola plant of the species Diplotaxis tenuifolia comprising a genetic determinant that leads to expression of resistance against Peronospora parasitica, which genetic determinant is obtainable from a Diplotaxis tenuifolia plant comprising said genetic determinant, representative seed of which was deposited with the NCIMB under deposit number NCIMB 41811.

2. Rucola plant as claimed in claim 1, obtainable by crossing a first rucola plant with a second rucola plant, wherein one of the said plants is grown from seed as deposited with the NCIMB under deposit number NCIMB 41811, or a progeny plant thereof, and selecting for a plant that shows resistance to Peronospora parasitica.

3. Rucola plant as claimed in claim 1 or 2,
wherein the genetic determinant is obtainable by introgression from a rucola plant, representative seed of which has been deposited with the NCIMB under deposit number NCIMB 41811.

4. Rucola plant as claimed in any of the claims 1-3, wherein the plant that comprises the genetic determinant is used as the female parent in a cross.

5. Seed comprising the genetic determinant as defined in any of the claims 1-4, wherein the plant that can be grown from the seed is resistant to Peronospora parasitica.

6. Progeny of a rucola plant as claimed in any of the claims 1-4, or of rucola seed as claimed in claim 5, which progeny is resistant to Peronospora parasitica.

7. Propagation material suitable for producing a plant as claimed in any one of the claims 1-4 or for producing seed as claimed in claim 5.

8. Propagation material as claimed in claim 7,
wherein the propagation material is selected from the group consisting of microspores, pollen, ovaries, ovules, embryos, embryo sacs, egg cells, cuttings, roots, root tips, hypocotyls, cotyledons, stems, leaves, flowers, anthers, seeds, meristematic cells, protoplasts, and cells..

9. Tissue culture of propagation material as claimed in claim 7 or 8.

10. Plant parts comprising leaves and stems of a rucola plant as claimed in any one of the claims 1-4 and 6.

11. Food product comprising plant parts, in particular leaves and stems of claim 10, or parts thereof, optionally in processed form.

12. Use of a plant as claimed in any one of the claims 1-4 and 6, or plants produced from the seed of claim 5, or from the propagation material as claimed in any one of the claims 7-9, as germplasm in a breeding program for the development of a rucola plant that shows resistance against Peronospora parasitica.
